(19) Europäisches Patentamt
European Patent Office
Office européen des brevets

(11) EP 2 036 488 A2

(12) EUROPEAN PATENT APPLICATION

(43) Date of publication:
18.03.2009 Bulletin 2009/12

(51) Int Cl.:
*A61B 5/00* (2006.01)

(21) Application number: 08016006.2

(22) Date of filing: 11.09.2008

(84) Designated Contracting States:
AT BE BG CH CY CZ DE DK EE ES FI FR GB GR
HR HU IE IS IT LI LT LU LV MC MT NL NO PL PT
RO SE SI SK TR
Designated Extension States:
AL BA MK RS

(30) Priority: 12.09.2007 JP 2007236475

(71) Applicant: Canon Kabushiki Kaisha
Tokyo (JP)

(72) Inventors:
• Nishihara, Hiroshi
Tokyo (JP)
• Masumura, Takahiro
Tokyo (JP)
• Yoshida, Hirofumi
Tokyo (JP)

(74) Representative: Weser, Wolfgang
Weser & Kollegen
Patentanwälte
Radeckestrasse 43
81245 München (DE)

(54) **Measurement apparatus**

(57) A measurement apparatus measures a spectroscopic characteristic and a structural characteristic of a specimen (E). At the measurement site of the specimen, a modulation of light from the light source part (1) by an acousto-optical effect and a generation of the ultrasound echo signal simultaneously occur, the light detecting means (5) detects modulated light (Iac) that is simultaneously generated, and the ultrasound detecting means (6) detects the ultrasound echo signal that is simultaneously generated.

FIG.1

EP 2 036 488 A2

**Description**

BACKGROUND OF THE INVENTION

Field of the Invention

The present invention relates to a measurement apparatus configured to measure a characteristic of a specimen (scattering medium).

Description of the Related Art

A conventional measurement apparatus as used for mammography measures a spectroscopic characteristic of an internal biological tissue. A conventional ultrasound echo apparatuses obtains a structural characteristic of a biological body. The medical diagnosis improves in quality and precision when a spectroscopic characteristic and a structural characteristic in a biological body are simultaneously measured and superposed.

The conventional spectroscopic measurement apparatuses apply Acousto-Optical tomography ("AOT") or Photo-Acoustic Tomography ("PAT"). AOT irradiates the coherent light and focused ultrasound into the biological tissue, and detects through a light detecting unit (a light detecting means) the modulated light as a result of an effect of light modulation (acousto-optical effect) in an ultrasound focusing area, as disclosed in U.S. Patent No. 6,738,653. On the other hand, PAT utilizes a difference in photo energy absorption rate between a measurement site, such as a tumor, and another tissue, and receives through an ultrasound detecting unit (an ultrasound detecting means) ultrasound (an acousto-optical signal) that occurs when the measurement site absorbs the irradiated photo energy and instantaneously expands.

Japanese Patent Laid-Open No. 2005-21380 is prior art that measures both a spectroscopic characteristic and a structural characteristic of a biological body using a PAT measurement apparatus and an ultrasound echo device, and receives a photo acoustic signal and an ultrasound echo signal by a common detecting device. U.S. Patent No. 6,264,610 arranges a near-infrared light source near a transducer configured to measure an ultrasound echo signal, and measures an ultrasound echo signal and a diffused light image generated by the near-infrared light source.

However, the prior art does not precisely correlate the spectroscopic characteristic with the structural characteristic, or the quality or the precision of the diagnosis does not necessarily improve. First of all, a specimen is typically a breast and is likely to deform. For this reason, when a separate ultrasound echo device is applied to the AOT measurement apparatus described in U. S. Patent No. 6, 738, 653 and the functional information and the structural information are separately measured, the specimen have different shapes in these measurements due to, for example, a pressure deformation by an ultrasound probe against a biological body. For this reason, it becomes difficult to precisely superpose two characteristics. Japanese Patent Laid-Open No. 2005-21380 uses a common device for the PAT detecting device and the ultrasound echo detecting device, and cannot simultaneously measure both characteristics. A time lag of measurements of both characteristics makes difficult to precisely superpose both characteristics because the specimen may move during the time lag. The spectroscopic characteristic measured by the apparatus of U.S. Patent No. 6,264,610 has a lower resolution than that measured by the apparatuses described in U.S. Patent No. 6, 738, 653 or Japanese Patent Laid-Open No. 2005-21380.

SUMMARY OF THE INVENTION

The present invention provides a measurement apparatus which can precisely correlate a spectroscopic characteristic with a structural characteristic with a fine resolution.

The present invention in its first aspect provides a measurement apparatus as specified in claims 1 to 6.

Further features of the present invention will become apparent from the following description of exemplary embodiments (with reference to the attached drawings).

BRIEF DESCRIPTION OF THE DRAWINGS

FIG. 1 is a block diagram of a measurement apparatus according to a first embodiment of the present invention.

FIG. 2 is a block diagram of a light source part in the measurement apparatus shown in FIG. 1.

FIG. 3 is a schematic perspective view of an ultrasound generating unit (an ultrasound source) in the measurement apparatus shown in FIG. 1.

FIG. 4 is a block diagram of an ultrasound focusing unit (an ultrasound focusing means) in the measurement apparatus shown in FIG. 1.

FIG. 5 is a block diagram of a light detecting unit and a first and a second signal processing units in the measurement apparatus shown in FIG. 1.

FIG. 6 is a timing chart for explaining an operation of the measurement apparatus shown in FIG. 1.

FIG. 7 is a graph which shows absorption spectra of $HbO_2$ and Hb in wavelengths between 600 and 1000nm.

DESCRIPTION OF THE EMBODIMENTS

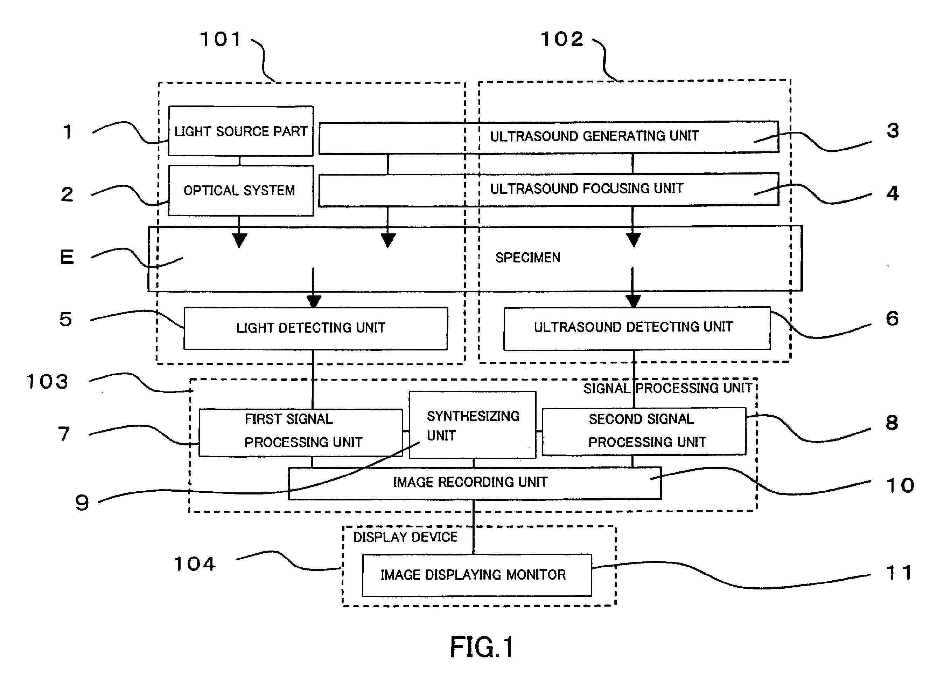

FIG. 1 is a block diagram of a measurement apparatus according to a first embodiment of the present invention. The measurement apparatus measures a spectroscopic characteristic and a structural characteristic of a specimen (scattering medium) E, and includes a spectroscopic characteristic measurement apparatus 101, an ultrasound echo measurement apparatus 102, a signal processing device 103, and a display device 104.

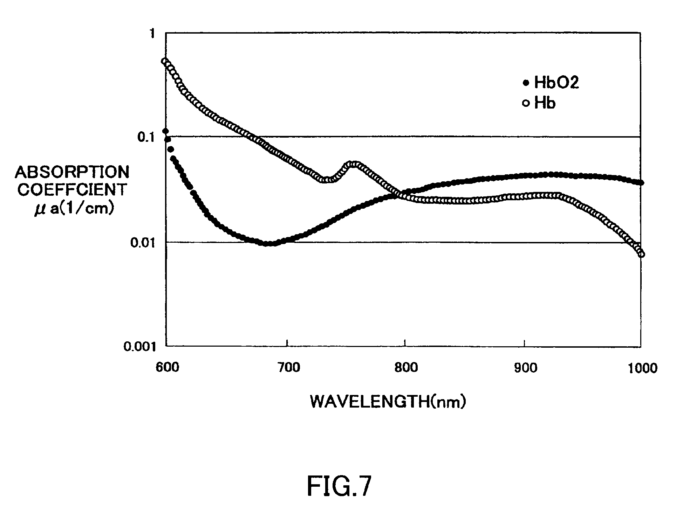

The specimen E is a biological tissue, such as a breast. It is known that a new blood vessel starts to form or that oxygen consumption increases as a tumor such as a cancer grows. Absorption spectroscopic characteristics of oxygenated hemoglobin ($HbO_2$) and deoxygenated hemoglobin (Hb) can be used to evaluate a formation of the new blood vessel or an increase of the oxygen consumption. FIG. 7 shows absorption spectra of $HbO_2$ and Hb in wavelengths between 600 and 1000nm.

The measurement apparatus measures Hb and $HbO_2$ concentrations in blood in a biological tissue based on the absorption spectra of $HbO_2$ and Hb of a plurality of wavelengths, and measures the Hb and $HbO_2$ concentrations at plural positions, thereby generating an image of the concentration distribution in the biological tissue and identifying areas of the new blood vessels. In addition, the measurement apparatus calculates the oxygen saturation degree based on the Hb and $HbO_2$ concentrations, and identifies an area that increases the oxygen consumption amount based on the oxygen saturation degree. The spectroscopic information of Hb and $HbO_2$ thus measured by the measuring apparatus can be used for diagnostics.

The spectroscopic characteristic measurement apparatus 101 measures a spectroscopic characteristic in a tissue of the specimen E by applying AOT. The spectroscopic characteristic measurement apparatus 101 includes a light source part 1, an optical system 2, an ultrasound generating unit 3, an ultrasound focusing unit 4, and a light detecting unit 5.

The light source part 1 is a light source which emits luminous fluxes having a plurality of wavelengths irradiated on the specimen E. The wavelength in the light source is selected among wavelengths in accordance with absorption spectra such as water, lipid, protein, oxygenated hemoglobin, and reduced hemoglobin. In an example, an appropriate wavelength falls upon a range between 600 to 1500 nm, because the light can highly transmit due to a small absorption of water that is a main ingredient of the internal biological tissue, and the spectra of the lipid, the oxygenated hemoglobin, and the deoxygenated hemoglobin are characteristic. The light source emits luminous fluxes of a continuous wave ("CW") having a constant intensity and a long coherent length (e. g. , equal to or longer than 1m). In a specific example, the light source may be comprised of a semiconductor laser or a wavelength-variable laser which generate various different wavelengths.

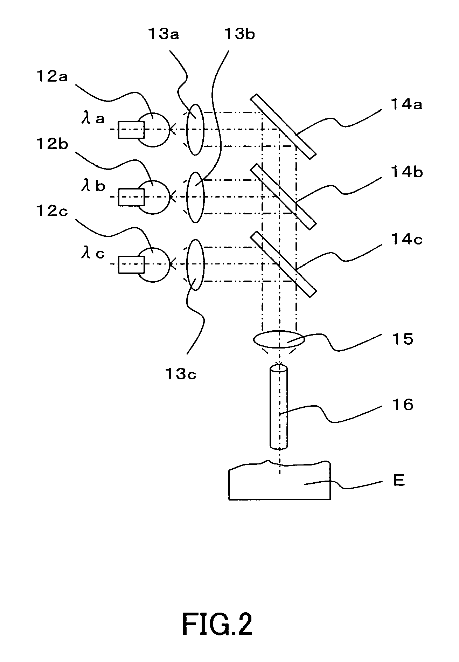

The optical system 2 guides the light from the light source part 1 to the specimen E. FIG. 2 represents an example of the optical system 2. In FIG. 2, the light source part 1 is comprised of semiconductor lasers 12a, 12b, and 12c having various different wavelengths. The lasers 12a, 12b, and 12c emit luminous fluxes of wavelengths $\lambda a$, $\lambda b$, and $\lambda c$ respectively. The optical system 2 includes lenses 13a, 13b, 13c, dichroic mirrors 14a, 14b, 14c, a focusing lens 15, and an optical fiber 16.

Each of the lenses 13a, 13b, and 13c collimates the luminous flux emitted from a corresponding one of the semiconductor lasers 12a, 12b, and 12c, and guides the collimated beam to a corresponding one of the dichroic mirrors 14a, 14b, and 14c. The dichroic mirror 14a reflects the light of the wavelength $\lambda a$, and the dichroic mirror 14b reflects the light of the wavelength $\lambda b$ and transmits the light of the wavelength $\lambda a$. The dichroic mirror 14c reflects the light of the wavelength $\lambda c$, and transmits the light of the wavelength $\lambda a$ and the light of the wavelength $\lambda b$. The light which reflects and transmits the dichroic mirrors 14a, 14b, and 14c is focused onto one end of the optical fiber 16. The optical fiber 16 guides the light to the specimen E. The light which passes through the optical fiber 16 is irradiated on the specimen E from the other end of the optical fiber 16.

The ultrasound generating unit 3 is an ultrasound transmitting device which transmits an ultrasound (an ultrasound pulse) to the specimen E. This embodiment sets ultrasound frequency to a range between 1 and several tens of MHz although the appropriate frequency may vary with a measurement depth or resolution of the specimen E in the ultrasound echo device.

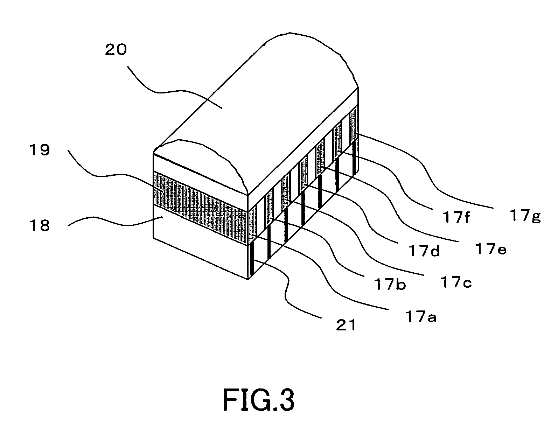

FIG. 3 is a schematic perspective view of a structure of a linear array search unit as an example of the ultrasound generating unit 3. A plurality of small reed shaped ultrasound transducers 17 are arranged on a backing member 18. An acoustic matching layer 19 is arranged on an ultrasound irradiating surface of the ultrasound transducers 17, and an acoustic lens 20 is arranged on the acoustic matching layer 19. A lead wire 21 is connected to each ultrasound transducer 17.

The ultrasound transducer 17 includes a piezoelectric element, which provides a piezoelectric effect of converting an applied voltage into ultrasound or of converting a received pressure change into a voltage. A device which converts ultrasound's mechanical oscillation into an electric signal or vice versa is referred to as an ultrasound transducer. The piezoelectric element may use a piezoelectric ceramic material as typified by lead zirconate titanate ("PZT"), or a polymer piezoelectric membrane material as typified by polyvinylidene-fluoride ("PVDF").

The backing member 18 absorbs an acoustic wave that propagates in a direction opposite to a traveling direction of the ultrasound, and restrains unnecessary oscillations of the ultrasound transducer 17. Since a piezoelectric element is significantly different from a biological body in acoustic impedance, a direct contact between the piezoelectric element and the biological tissue causes reflections on the interface to be too large to efficiently transmit (or receive) the ultrasound. For this reason, the acoustic matching layer 19 made of a material having an intermediate acoustic impedance is inserted into a space between the ultrasound transducer 17 composed of the piezoelectric element and the biological body so as to efficiently transmit the ultrasound.

The acoustic lens 20 restrains spreading of the ultrasound in an orthogonal direction to the arrangement direction of the ultrasound transducer 17. The lead wire 21 is used to transmit and receive a signal of the ultrasound transducer 17.

The ultrasound focusing unit 4 focuses the ultrasound from the ultrasound generating unit 3 onto the measurement site X in the specimen E. An ultrasound focusing method may use a spherical, cylindrical, or aspheric concave ultrasound transducer, or an acoustic lens, electronic focusing that utilizes an array search unit. In the concave ultrasound transducer, a curvature of the concave surface determines a focusing position. The acoustic lens is a convex lens when made of a material having a sonic velocity lower than that in the biological tissue and, like the concave ultrasound transducer, a curvature of the convex surface determines a focusing position.

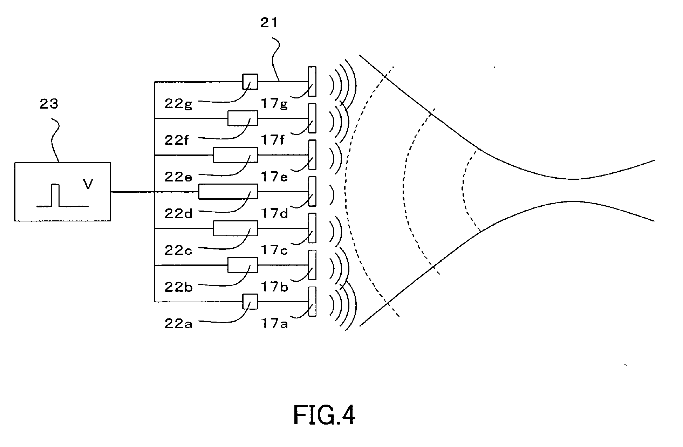

This embodiment uses electronic focusing that uses the above array search unit. Referring now to FIG. 4, a description will be given of this illustration. FIG. 4 is a block diagram as an example of the ultrasound focusing unit 4.

Variable delay elements 22a, b, c, d, e, f, and g and a pulsar 23 are respectively connected to a plurality of the arranged ultrasound transducers 17a, b, c, d, e, f, and g via the lead wire 21. The variable delay element 22 uses a member that winds a coil-shaped thin electric wire to delay a transmission of an electric signal that transmits through the electrical wire. A delay time period of the electronic signal is adjustable by switching a plurality of taps which are provided in the middle of the coil. The pulsar 23 is a device that generates a pulse voltage applied to the ultrasound transducer 17.

When the variable delay element 22 closer to the center has a longer delay time period ($\tau a = \tau g < \tau b = \tau f < \tau C = \tau e < \tau d$), a synthesis wavefront formed by each ultrasound transducer 17 becomes a focusing wavefront. Thus, control over a delay time period given by the variable delay element 22 can provide control over an ultrasound focusing position. The similar control can also provide control over a traveling direction of the ultrasound.

FIG. 4 illustrates the ultrasound transmissions, but a similar relationship is true of the reception, and the variable delay elements 22 can corrects a difference of the distance between a ultrasound echo generating source and each ultrasound transducer 17 so as to equalize the same phases. Electrical focusable search units, other than the linear array search unit, include a 2D array search unit which arranges ultrasound transducers on a two-dimensional surface, and an annular array search unit which concentrically arranges ring-shaped transducers. In the ultrasound focusing unit 4 using a concave ultrasound transducer or an acoustic lens, the focusing position of the ultrasound can be controlled by changing a position of the ultrasound focusing unit 4 through mechanical driving.

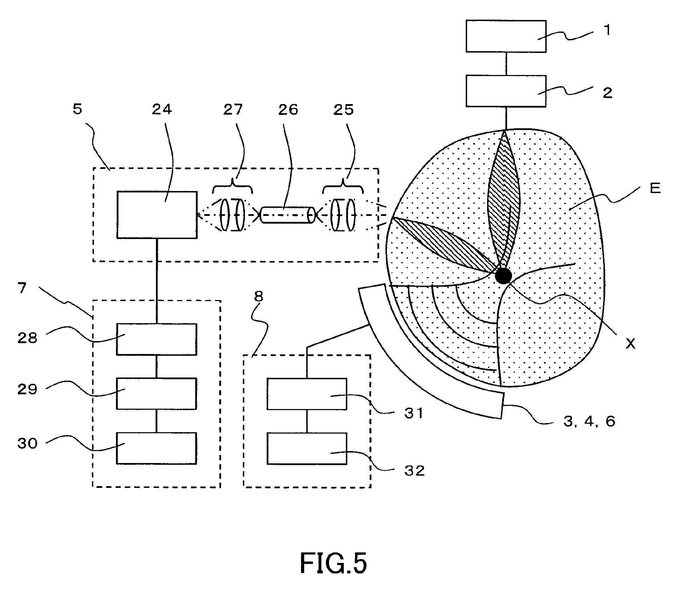

The light detecting unit 5 detects the light that has propagated in the tissue of the specimen E and exited to the outside. The light detecting unit 5 is comprised of a light sensor 24, a lens 25, an optical fiber 26, and a lens 27, as shown in FIG. 5. Here, FIG. 5 is a schematic block diagram of one example of the light detecting unit 5.

As shown in FIG. 5, the light emitted from the light source part 1 enters the specimen E via the optical system 2. The light incident upon the specimen E repeats absorptions and scatters inside the specimen E several times, and then propagates in various directions. The propagation of the light in the absorption-scattering medium can be described by a light diffusion equation. Assume that φ (rs) is a fluence rate of a photon of the light's propagation from the light source part 1 to the ultrasound focusing position (the measurement site) X shown in FIG. 5, and φ (rd) is a fluence rate of a photon of the light's propagation from the ultrasound focusing position X to the light detecting unit 5.

The acoustic pressure increases near the ultrasound focusing position X, changes the density and the refractive index in the absorption-scattering medium, and displaces the absorption-scattering medium. When the light passes through the ultrasound focusing area, an optical phase of the light changes due to a change of the refractive index and a displacement of the absorption-scattering medium. The acoustic pressure locally increases at the focusing position X, and the focusing position X is subject to the influence of the ultrasound (such as the change of the refractive index and the displacement of the absorption-scattering medium) more strongly than the peripheral part. Thus, a larger amount of the modulated light that is modulated by the ultrasound with a frequency Q (MHz) is likely to occur at the position than in its peripheral areas. An optical signal generated from the ultrasound focusing area can be selectively measured by selectively detecting the modulated light caused by the acousto-optical effect.

Assume that m is a modulation depth by which the light is modulated by ultrasound, and I0 is an intensity of an incident light. Then, a detected light signal Iac is given as follows:

EQUATION 1

$$Iac = I0 \cdot \Phi(rs) \cdot m \cdot \Phi(rd)$$

The light sensor 24 detects both the modulated light Iac modulated by the ultrasound, and the multi-scattering, non-modulated light that is free of the ultrasound modulation. The light sensor 24 can measure a light signal at a desired position by controlling (or scanning) the ultrasound focusing position X through the ultrasound generating unit 3 and the ultrasound focusing unit 4. The light sensor 24 may apply a photoelectric conversion element, such as a photomultiplier ("PMT"), a charge coupled device ("CCD"), and a complementary metallic oxidized film semiconductor ("CMOS"). However, the selected light sensor needs to have a sufficient sensitivity to the light having a wavelength in a range between 600 and 1500 nm generated by the light source part 1.

The lens 25 focuses the light that has propagated in the tissue of the specimen E and exited to the outside, and guides the light to the optical fiber 26. The lens 27 guides the light that has exited from the optical fiber 26 to the light sensor 24. The signal detected by the light sensor 24 is transmitted to the first signal processing unit 7.

The ultrasound echo measurement apparatus 102 measures a structural characteristic in a tissue of the specimen E by using an ultrasound echo. The ultrasound echo measurement apparatus 102 includes the above ultrasound generating unit 3 as means for transmitting the ultrasound, the above ultrasound focusing unit 4, and an ultrasound detecting unit 6. In this way, the ultrasound generating unit 3 is commonly used for the spectroscopic characteristic measurement apparatus 101 and the ultrasound echo measurement apparatus 102. The ultrasound focusing unit 4 is commonly used for the spectroscopic characteristic measurement apparatus 101 and the ultrasound echo measurement apparatus 102.

The ultrasound detecting unit 6 serves as an ultrasound receiving device which receives an ultrasound echo signal generated from the internal tissue of the specimen E. It is composed of a piezoelectric element as well as the ultrasound generating unit 3 and, in the example of the above search unit, one device can provide both transmitting and receiving functions (the ultrasound transducer).

The signal processing device 103 generates an image by processing a signal which includes the spectroscopic characteristic and is measured by the spectroscopic characteristic measurement apparatus 101 and a signal which includes the structural characteristic and is measured by the ultrasound echo measurement apparatus 102. The signal processing device 103 includes a first signal processing unit 7, a second signal processing unit 8, a synthesizing unit 9, and an image recording unit 10.

The first signal processing unit 7 generates an image of the spectroscopic characteristic of the measurement site in the specimen E. The first signal processing unit 7 shown in FIG. 5 is comprised of a filter 28, a signal analyzing device 29, an image generating device 30. The filter 28 separates the modulated light Iac from the non-modulated light, and measures the modulated light Iac. The filter 28 may apply a band pass filter which selectively detects a signal having a specific frequency or a lock-in amplifier which detects by amplifying the light of a specific frequency. The signal analyzing device 29 produces distribution data of a spectroscopic characteristic in the specimen E based on coordinate data of the focused ultrasound and the light signal Iac corresponding to the coordinate data.

The second signal processing unit 8 generates an image of the structural characteristic of the measurement site in the specimen E. The second signal processing unit 8 shown in FIG. 5 comprises a signal analyzing device 31 and an image generating device 32. The signal analyzing device 31 calculates the structural characteristic of the internal tissue based on the ultrasound echo signal generated from the ultrasound pulse of a frequency $\Omega$ (MHz) that has been irradiated by the ultrasound generating unit 3 and the ultrasound focusing unit 4 onto the position X of the internal tissue of the specimen E. The image generating device 32 generates an image based on a distribution of a structural characteristic calculated by the signal analyzing device 31.

The signal processing unit 9 produces an image which synthesizes the image of the spectroscopic characteristic generates by the first signal processing unit 7 and the image of the structural characteristic generated by the second signal processing unit 8. The synthesizing unit 9 synthesizes the two characteristics while correlating the measurement position X of the spectroscopic characteristic with the measurement position X of the structural characteristic. Further, the synthesizing unit 9 enables the image of the spectroscopic characteristic to be distinguished from the image of the structural characteristic in different colors.

The image recording unit 10 records the image of the spectroscopic characteristic generated by the first signal processing unit 7, the image of the structural characteristic generated by the second signal processing unit 8, and the synthesized image generated by the synthesizing unit 9. The image recording unit 10 may use a data recording device such as an optical disc, a magnetic disc, a semiconductor memory, and a hard disc drive.

The display device 104 displays an image generated by the signal processing device 103, and has an image display monitor 11. The display device 104 displays the image of the spectroscopic characteristic generated by the first signal processing unit 7, the image of the structural characteristic generated by the second signal processing unit 8, and the synthesized image generated by the synthesizing unit 9. The image display monitor 11 can use a display device such as a liquid crystal display, a CRT, and an organic EL.

The measurement apparatus measures the spectroscopic characteristic and the structural characteristic of the internal tissue of the specimen E, and displays the generated synthesized image that precisely superposes both characteristics.

In operation of the spectroscopic characteristic measurement apparatus 101 in the measurement apparatus, the light source part 1 emits the light having a specific wavelength, and the optical system 2 irradiates the light on the specimen E. More specifically, the semiconductor lasers 12a to c in the light source part 1 generate CW luminous fluxes having the wavelengths λa to c. The lenses 13a to c, the dichroic mirrors 14a to c, the lens 15, and the optical fiber 16 in the optical system 2 irradiate the light onto the specimen E. Next, the ultrasound focusing unit 4 focuses the ultrasound pulse having the frequency Ω (MHz) through electronic focusing, which is transmitted from the ultrasound generating unit 3, onto a specific position (the measurement site) X in the internal tissue of the specimen E. As a result, the acoustic pressure at the focusing position X becomes higher than at the peripheral area, and the light irradiated onto the focusing position X is turned to the modulated light Iac by the acousto-optical effect. Then, the light sensor 24 in the light detecting unit 5 detects the modulated light Iac and non-modulated light that are emitted from the specimen E via the lens 25, the optical fiber 26, and the lens 27.

In operation of the ultrasound echo measurement apparatus 102 in the measurement apparatus, the ultrasound focusing unit 4 focuses the ultrasound pulse which is transmitted from the ultrasound generating unit 3, onto the specific position (the measurement site) X in the internal tissue in the specimen E. Next, the ultrasound detecting unit 6 detects the ultrasound echo signal in the specimen E generated by the ultrasound pulse.

In this way, the ultrasound generating unit 3 and the ultrasound focusing unit 4 are commonly used in the measurement apparatus. This configuration can not only provide a smaller and less expensive measurement apparatus as a result of that the spectroscopic characteristic measurement apparatus 101 and the ultrasound echo measurement apparatus 102 share some components, but also simultaneously measure the spectroscopic characteristic and the structural characteristic. Thus, the ultrasound pulse generated by the ultrasound generating unit 3 is used to simultaneously measure both the spectroscopic characteristic and the structural characteristic. When the ultrasound focusing unit 4 focuses the ultrasound pulse generated by the ultrasound generating unit 3, onto the measurement site X, a modulation of the light from the light source part 1 due to the acousto-optical effect (the generation of the modulated light Iac) and a generation of the ultrasound echo signal simultaneously occur on the measurement site X. Then, the light detecting unit 5 generates the modulated light Iac that is simultaneously generated, and the ultrasound detecting unit 6 detects the ultrasound echo signal that is simultaneously generated. The modulated light reaches the ultrasound detecting unit 5 at the light velocity, and the ultrasound echo signal reaches the ultrasound detecting unit 6 at the sonic velocity. Thus, arrival time is different between them. Nevertheless, since the modulation of the light and the generation of the ultrasound echo signal on the same measurement site simultaneously occur, the spectroscopic characteristic and the structural characteristic can be precisely correlated to one another.

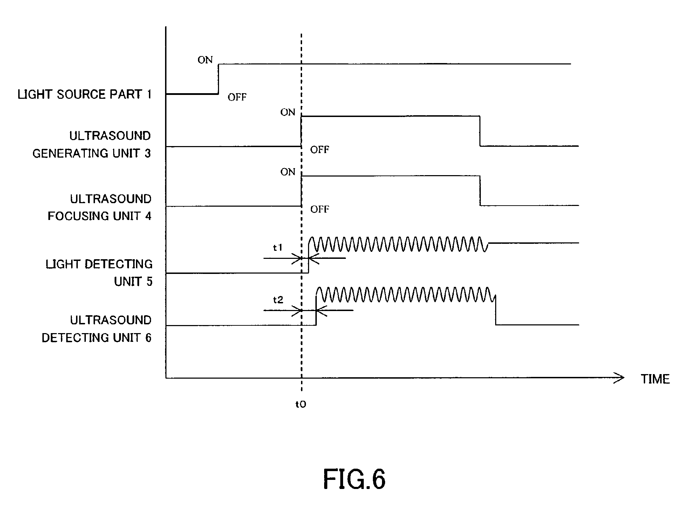

Referring now to FIG. 6, a description will be given of this phenomenon. FIG. 6 is a chart which shows the time of detecting a light signal and an echo signal from the generation of the ultrasound pulse. In this figure, when the light is irradiated onto the specimen E from the light source part 1, the ultrasound generating unit 3 and the ultrasound focusing unit 4 generate the ultrasound pulse at a time t0. Where t1 is a time period by which the ultrasound pulse is generated and reaches the position X, the modulated light signal and the echo signal at the position X are almost simultaneously generated t1 after t0. Since the light emitted by the light source 1 is sufficiently higher than the ultrasound speed, the light detecting unit 5 detects the modulated light signal for a time period from about t1 after t0 to time at which the ultrasound pulse is applied. On the other hand, where t2 is a time period from when the ultrasound pulse is generated to when the echo signal at the position X reaches the ultrasound detecting unit 6, the ultrasound detecting unit 6 detects the echo signal for a time period from about t2 to t0 to time at which the ultrasound pulse is applied. In this way, the modulated light signal and the echo signal detected in this embodiment are simultaneously generated at the same position X by the same ultrasound pulse, and spatial difference and time difference are extremely small.

Next, the first signal processing unit 7 separates the modulated light having the frequency Ω (MHz) from light signals of both the modulated light Iac and the non-modulated light that are detected by the light sensor 24 by the filter 28. The first signal processing unit 7 generates an image of the spectroscopic characteristic of the internal tissue in the specimen E based on the light intensity and the phase. More specifically, the signal analyzing unit 29 produces distribution data of the spectroscopic characteristic in the specimen E based on coordinate data of the focused ultrasound and the separated modulated signal corresponding to the coordinate data. The image generating device 30 generates an image from the distribution data of the spectroscopic characteristic in the specimen E generated by the signal analyzing unit 29.

The second signal processing unit 8 generates an image of the structural characteristic of the internal tissue in the specimen E based on the ultrasound echo signal. More specifically, the signal analyzing device 31 in the second signal processing unit 8 calculates the structural characteristic of the measurement site based on the ultrasound echo signal detected by the ultrasound detecting unit 6. The image generating device 32 generates an image based on the distribution of the structural characteristic calculated by the signal analyzing device 31.

Next, the synthesizing unit 9 synthesizes the spectroscopic characteristic with the structural characteristic for each position in the specimen E, and displays the image on the image display monitor 11. The synthesizing unit 9 synthesizes the image of the spectroscopic characteristics generated by the first signal processing unit 7 with the image of the structural characteristic generated by the second signal processing unit 8 while correlating the measurement position X of the spectroscopic characteristic with the measurement position X of the structural characteristic. Further, the image of the spectroscopic characteristic and the image of the structural characteristic can be made distinguished from one another in different colors. The image of the spectroscopic characteristic generated by the first signal processing unit 7, the image of the structural characteristic generated by the second signal processing unit 8, and the synthesized image generated by the synthesizing unit 9 are displayed on the image display monitor 11 in the display device 104 and recorded in the image recording unit 10.

As described above, the ultrasound pulse used to measure the spectroscopic characteristic and the structural characteristic is irradiated on the same measurement site X at the same time, and a special difference and a time difference are extremely small. For this reason, this embodiment can measure both characteristics almost at the same time. Since the influence of an examinee's movement and a measurement time difference are small, the images generated from both characteristics can be superposed with a high precision, and this image improve the precision and the quality of the diagnosis. The spectroscopic characteristic on the measurement site on which the ultrasound is focused is measured with a fine resolution by applying AOT. Since the ultrasound generating unit 3 and the ultrasound focusing unit 4 are commonly used, a smaller and less expensive measurement apparatus can be provided.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

**Claims**

1. A measurement apparatus comprising:

    a spectroscopic characteristic measurement apparatus (101) which includes a light source part (1) and a light detecting means (5), and measures a spectroscopic characteristic of a specimen (E) by applying acousto-optical tomography; and
    an ultrasound echo measurement apparatus (102) which includes an ultrasound detecting means (6), and measures a structural characteristic of the specimen by applying an ultrasound echo signal,
    wherein each of the spectroscopic characteristic measurement apparatus and the ultrasound echo measurement apparatus further includes:
    an ultrasound source (3) which is commonly arranged in the spectroscopic characteristic measurement apparatus and the ultrasound echo measurement apparatus, and transmits an ultrasound pulse to the specimen; and
    a ultrasound focusing means (4) which is commonly arranged in the spectroscopic characteristic measurement apparatus and the ultrasound echo measurement apparatus, and focuses the ultrasound pulse transmitted by the ultrasound source onto a measurement site (X) of the specimen,
    **characterized in that** at the measurement site of the specimen, a modulation of light from the light source part by an acousto-optical effect and a generation of the ultrasound echo signal simultaneously occur, the light detecting means detects modulated light (Iac) that is simultaneously generated, and the ultrasound detecting means detects the ultrasound echo signal that is simultaneously generated.

2. A measurement apparatus according to claim 1, further comprising:

    a first signal processing means (7) configured to generate an image of a spectroscopic characteristic of the measurement site in the specimen;
    a second signal processing means (8) configured to generate an image of a structural characteristic of the measurement site in the specimen; and
    a synthesizing means (9) configured to generate a synthesized image that synthesizes the image of the spectroscopic characteristic with the image of the structural characteristic.

3. A measurement apparatus according to claim 2, wherein the synthesizing means enables the image of the spectroscopic characteristic to be distinguished from the image of the structural characteristic by in different colors.

4. A measurement apparatus according to any one of claim 2 or 3, further comprising an image recording means (10) configured to record the image of the spectroscopic characteristic generated by the first signal processing means, the image of the structural characteristic generated by the second signal processing means, and a synthesized image generated by the synthesizing means.

5. A measurement apparatus according to any one of claims 2 to 4, further comprising a display device (104) configured to display the image of the spectroscopic characteristic generated by the first signal processing means, the image of the structural characteristic generated by the second signal processing means, and a synthesized image generated by the synthesizing means.

6. A measurement apparatus according to claim 1, wherein the ultrasound source and the ultrasound detecting means are integrated into one ultrasound transducer (17).

FIG.1

FIG.2

FIG.3

FIG.4

FIG.5

FIG.6

FIG.7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6738653 B
- JP 2005021380 A
- US 6264610 B